# EUROPÄISCHE PATENTSCHRIFT

(11) **EP 1 149 172 B1**
(45) Veröffentlichungstag und Bekanntmachung des Hinweises auf die Patenterteilung: **26.10.2005**
(21) Anmeldenummer: 00901110.7
(22) Anmeldetag: 17.01.2000
(51) Int. Cl.: C12Q 1/04, B01D 21/26, C12N 1/02, C02F 1/38

(54) **VERFAHREN ZUR BESTIMMUNG DER MIKROBIELLEN KONTAMINATION**
METHOD FOR DETERMINING MICROBIAL CONTAMINATION
PROCEDE POUR DETERMINER UNE CONTAMINATION MICROBIENNE

(30) Priorität: 02.02.1999 DE 19904057
(43) Veröffentlichungstag der Anmeldung: 31.10.2001
(73) Patentinhaber: OMYA AG, 4665 Oftringen (CH)
(72) Erfinder: BURI, Matthias, CH-4852 Rothrist (CH); SCHWARZENTRUBER, Patrick, CH-4656 Starrkirch-Wil (CH)
(74) Vertreter: Behnisch, Werner
(86) Internationale Anmeldenummer: PCT/EP2000/000328
(87) Internationale Veröffentlichungsnummer: WO 2000/046392

(56) Entgegenhaltungen:
- EP-A- 0 724 902
- EP-A- 0 816 512
- WO-A-91/04318
- DE-C- 19 532 802
- DATABASE WPI Section Ch, Week 199118 Derwent Publications Ltd., London, GB; Class D16, AN 1991-130876 XP002901039 & SU 1 571 060 A (PROTEIN BIOSYNTHESI), 15. Juni 1990 (1990-06-15)

## Beschreibung

Die vorliegende Erfindung betrifft ein Verfahren zur quantitativen und/oder qualitativen Bestimmung der mikrobiellen Kontamination von wäßrigen, Mineralien und/oder Pigmenten und/oder Füllstoffen und/oder Fasermaterialien, wahlweise in Kombination mit Polymeren in kolloidaler Form enthaltenden Suspensionen, Emulsionen oder Dispersionen.

Die Bestimmung von Verkeimung und Hygiene-Kontrollen von wäßrigen Suspensionen mit herkömmlichen Methoden sind im wesentlichen alle an die Verrnehrungsfähigkeit der nachzuweisenden Mikroorganismen gebunden. Diese Verfahren benötigen naturgemäß Zeit, die zwischen 24 h und mehreren Tagen liegen kann. Für viele Fragestellungen sind diese Zeiträume zu groß, und die Ergebnisse liegen zu spät vor, um in Produktionsprozessen noch lenkend eingreifen zu können. Besonders für die Kontrolle vor dem Versand werden immer wieder Verfahren gefordert, die sich durch ein kurzes Untersuchungsintervall auszeichnen.

So ist beispielsweise das grundlegende Problem der herkömmlichen mikrobiologischen Analysen von aeroben mesophilen Keimen, wie Plate Count oder Easicult, die lange Inkubationszeit von bis zu 48 h. Eine Auswertung und somit eine Bestimmung der Keimzahl ist bei diesen Methoden vor zwei Tagen nicht möglich. Hinzu kommen noch verschiedene andere Einflüsse wie z.B. Nährboden, Partialdruck des Sauerstoffs (aerob/-anaerob), Selektivität, pH und noch vieles mehr.

Es ist deshalb oft nicht möglich, die Keimzahl von Produkten wie Pigmentslurries vor Auslieferung zum Kunden zu bestimmen und durch Zusatz von biozid wirkenden Substanzen lenkend einzugreifen. Durch die langen Transportwege von bis zu 6 Wochen im Hochseeschiff oder der Bahn kann die Pigmentslurry jedoch verderben und unbrauchbar werden. Die weisse Pigmentslurry kann sich grau verfärben und zu stinken beginnen Die preventive Überdosierung von biozid wirkenden Substanzen zur Pigmentslurry, die heutige einzige Möglichkeit um einen Verderb auszuschliessen ist sehr kostenintensiv, eine Verschleuderung von Resourcen und ökologisch unsinnig. Im weiteren müssen zur Analyse von aeroben mesophilen Keimen und Pilzen zwei verschiedene Ansätze gemacht werden. Weiterhin sind Verdünnungsreihen erforderlich, was die Anzahl der Analysen um ein mehrfaches erhöht.

Gängige Methoden zur Bestimmung von Keimen in der Papier- und Pigmentindustrie sind beispielsweise in Schweizerisches Lebensmittelbuch, Kapitel 56, Abschnitt 7.01, Ausgabe 1985, Revision 1988, "Bestimmung von aeroben Bakterien und Keime" und in Schweizerisches Lebensmittelbuch, Kapitel 56, Abschnitt 7.22, Ausgabe 1985, Revision 1988, "Bestimmung von Pilzen" beschrieben. Üblicherweise beträgt die Inkubationszeit, bevor eine Bestimmung durchgeführt werden kann, je ca. 48 Stunden.

Von der Firma Microbial Systems Ltd. wurde der Cellfacts ^{R} -Teilchenanalysen-Vorrichtung und -Verfahren entwickelt. Nähere Informationen hierüber befinden sich Labor flash 9/96, Zeitung mit Leserdienst für Labor und Forschung, Ott Verlag + Druck AG, Ch-3607 Thun, Schweiz. So wird beispielsweise angegeben, daß der Cellfacts-Analysator auch zur Bestimmung der Anzahl der Keime in Calciumcarbonat-Aufschlämmungen einsetzbar ist. Im Labor der Anmelderin durchgeführte Untersuchungen zeigten jedoch, daß eine derartige Bestimmung in der vom Hersteller beschriebenen Weise nicht oder nur ungenau möglich ist.
Das Meßprinzip des Cellfacts-Analysators beruht auf der Messung von Bakterien, Pilzen und Hefen als Teilchen in einem elektrischen Feld, wobei durch intervallweises Inkubieren der Proben und nachfolgender Messung die Zunahme der Anzahl an Teilchen bestimmt wird und bei exponentiellem Wachstum auf tₒ zurück extrapoliert wird. Dieses Meßprinzip funktioniert auch bei einer "niedrigen" Anzahl an "Fremdteilchen" mit gleichen oder ähnlichen Größen wie die Bakterien, Pilze und Hefen. Bei Suspensionen und Emulsionen mit einem Anteil an "Fremdteilchen", also von Mineralien, Füllstoffen und/oder Pigmenten, von > 1 Gew.% ist die Anzahl der inerten, in der gleichen Größe wie die Mikroorganismen vorliegenden "Fremdteilchen" von 0,5 - 20 µm, also der Blindwert tₒ , derart hoch, daß eine weitere Zunahme von Keimen durch Vermehrung über eine Zeitspanne von < 10 Stunden im Inkubator nicht mehr detektierbar ist. Das Cellfacts-Analysegerät kann so nicht mehr hinreichend genau messen, und der Hersteller fordert, um eine Anwendbarkeit sicherzustellen, eine Verdünnung der Ausgangslösung.

Eine Zunahme von 10³ Teilchen/ml , bei einem Blindwert von 10⁸ Teilchen/ml, ist durch das Cellfacts-Analysegerät nicht mehr signifikant erfaßbar. Die geforderte Verdünnung aufgrund der "Fremdteilchen" ist jedoch so groß, daß die Verdünnung der vermehrungsfähigen Organismen, die dadurch natürlich um den gleichen Faktor verdünnt werden, nicht mehr signifikant und das erhaltene Resultat falsch ist. "Fremdteilchen" mit einem Durchmesser von > 20 µm können zudem die Meßzelle von nur 30µm Durchmesser verstopfen. Die "Fremdteilchen" können beispielsweise mineralischer Natur sein wie Calciumcarbonat, synthetischer organischer polymerer Natur wie Polystyrolacrylat-Dispersionen oder natürlicher organischer Natur wie Stärkelösungen oder Hemizellulosen und/oder Cellulosefasern oder eine Kombination aus den obigen Teilchen, wie sie beispielsweise im Kreislauf einer Papierfabrik auftritt.

Es ist eine Aufgabe der vorliegenden Erfindung, ein schnelles und leistungsfähiges, einfach durchführbares Verfahren zur quantitativen und/oder qualitativen Bestimmung der mikrobiellen Kontamination von wäßrigen, Mineralien und/oder Pigmente und/oder Füllstoffe und/oder Fasermaterialen enthaltenden Suspensionen, Emulsionen oder Dispersionen bereitzustellen, das die oben beschriebenen Nachteile des Standes der Technik vermeidet.

Diese Aufgabe wird erfindungsgemäß durch ein Verfahren zur quantitativen und/oder qualitativen Bestimmung der mikrobiellen Kontamination von Mineralien und/oder Pigmente und/oder Füllstoffe und/oder Fasemlaterialien enthaltenden Suspensionen, Emulsionen oder Dispersionen gelöst, welches dadurch gekennzeichnet ist, daß
a) eine Probe der Suspensionen, Emulsionen oder Dispersionen mit einer oder mehreren durch Mikroorganismen abbaubaren, als Trennmittel zwischen den Mikroorganismen und den Mineralien, Pigmenten, Füllstoffen und/oder Fasermaterialien wirkenden, organischen Substanzen vermischt wird, wobei die Menge an bioabbaubarer Substanz so gewählt wird, daß eine Abtrennung der Mikroorganismen und der Mineralien, Füllstoffe, Pigmente und/oder Fasermaterialien ermöglicht wird, und wahlweise zur Erhöhung der Anzahl der Mikroorganismen inkubiert wird,
b) die so erhaltene Mischung zentrifugiert wird, um die Mineralien und/oder Füllstoffe und/oder Pigmente und/oder Fasermaterialien mehrheitlich von den Mikroorganismen abzutrennen, wobei sich die Mikroorganismen in der oberen Phase befinden, und
c) der durch die Zentrifugation erhaltene wässrige Überstand bevorzugt einer Inkubation oder mehreren Inkubationen unterworfen wird, um die Anzahl der Mikroorganismen in diesem Überstand zu vermehren, und
d) die Anzahl und/oder Größe und/oder Art der Mikroorganismen im Überstand bestimmt wird.

Bevorzugte Ausgestaltungen der Erfindung ergeben sich aus den Unteransprüchen sowie der nachfolgenden Beschreibung und den Ausführungsbeispielen.

Die quantitative und qualitative Bestimmung von Mikroorganismen gehört seit langem zum Stand der Technik. Besondere Schwierigkeiten bereitet jedoch die Bestimmung der mikrobiellen Kontamination dann, wenn eine hohe Konzentration von weiteren Feststoffteilchen wie Mineralien, Pigmenten, Füllstoffen und/oder Fasermaterialien in der zu untersuchenden Probe vorliegt. Da diese "Fremdteilchen" häufig eine ähnliche Größe wie die Mikroorganismen aufweisen, ist üblicherweise nur durch eine hohe Verdünnung der Ausgangsprobe die Anzahl der "Fremdteilchen" zu minimieren. Durch diese hohe Verdünnungsstufen wird jedoch gleichzeitig und unvermeidlich die Anzahl der kontaminierenden Mikroorganismen reduziert, und es ist ein langer Inkubationszeitraum notwendig, um die Anzahl der Mikroorganismen durch Vermehrung so zu erhöhen, das eine sichere Detektion ermöglicht wird.

Es bestand deshalb die Notwendigkeit, ein neues Verfahren der eingangs erwähnten Art zu schaffen, das in wesentlich kürzerer Zeit reproduzierbare und verläßliche Ergebnisse bezüglich der Anzahl, Größe und/oder Art der Mikroorganismen in der untersuchten Probe liefert.

Überraschend und nicht vorhersehbar wurde gefunden, daß durch die Zugabe abbaubarer organischer Substanzen zur Probe und eine nachfolgende Zentrifugation die Mikroorganismen vom inerten Material ("Fremdteilchen") abtrennbar sind. Üblicherweise haften die Mikroorganismen nämlich bevorzugt an den Mineral-, Pigment-, Füllstoff- und/oder Fasermaterialien an der Oberfläche an und lassen sich nur sehr schwer von der Oberfläche abtrennen. Durch einen einfachen Zentrifugationsschritt sedimentieren zwar die "Fremdteilchen", ziehen jedoch dadurch, daß die Mikroorganismen bevorzugt an diesen Teilchen haften, diese mit in das Sediment, so daß der im Überstand verbleibende Mikroorganismenanteil falsche Werte über den Grad der Verkeimung mit Mikroorganismen liefert.

Erstaunlicherweise konnte gezeigt werden, daß durch die Zugabe von biologisch abbaubaren organischen Substanzen eine Trennung der Mikroorganismen und der Mineralien, Pigmente, Füllstoffe und/oder Fasermaterialien erreicht wird. Bevorzugt ist die durch Mikroorganismen abbaubare organische Substanz ein Nährmedium, das üblicherweise zur Kultivierung der Mikroorganismen eingesetzt wird. Dieses Nährmedium wirkt überraschenderweise als Trennmittel zwischen den Mikroorganismen und den Fremdpartikeln und ermöglicht so überhaupt erst die Abtrennung und Auftrennung beider und stört die weiteren Schritte in der Analyse der Mikroorganism nicht. Nicht, oder nur schwer biologisch abbaubare Stoffe bergen die Gefahr, zwar die Mikroorganismen von den inerten Teilchen zu trennen, aber zugleich hemmend aufs Wachstum von Mikroorganismen zu wirken und somit ein falsches Resultat zu bewirken.

Durch das erfindungsgemäße Verfahren kann nicht nur ein Arbeitsschritt, nämlich der Verdünnungsschritt, eingespart werden, sondern zudem wird auch die Inkubationszeit außerordentlich verkürzt. Weiterhin wird nicht irgendein Trennmittel zugegeben, sondern das Trennmittel wirkt gleichzeitig als Nährlösung, die optimalerweise zur Vermehrung der zu untersuchenden Mikroorganismen einsetzbar ist und in einer bevorzugten Ausführungsform so gewählt wird, daß sie für eine bestimmte, zu untersuchende Mikroorganismenart, beispielsweise für Bakterien, Pilze oder Hefen, spezifisch ist.

Erst durch die Einführung einer durch Mikroorganismen abbaubaren organischen Substanz, bevorzugt in Form einer Nährlösung bzw. eines Nährmediums, und durch die Einführung des Zentrifugationsschrittes wird die erfindungsgemäß gestellte Aufgabe gelöst. Die Analysenzeit wird verkürzt, da in der zu untersuchenden Probe eine geringere Anzahl an "Fremdteilchen" enthalten ist oder sie wird überhaupt erst ermöglicht. Weiterhin kann die Ausgangszahl und der Zuwachs an mikrobiologischen Teilchen durch Inkubation über die Zeit geringer sein als bei Proben mit einem höhen Blindwert, also einer höheren Anzahl an "Fremdteilchen".

Durch das erfindungsgemäße Verfahren können wäßrige Suspensionen, Emulsionen und Dispersionen untersucht werden, die, unter anderem, Mineralien, Pigmente, Füllstoffe und/oder Fasermaterialien, beispielsweise Cellulosefasern und wahlweise weiterhin Polymere, z.B. natürliche, synthetische oder halbsynthetische Polymere in kolloidaler Form, enthalten. Beispiele für derartige Polymere sind: Styrolbutadien, Styrolacrylat, Melaminharze, Formaldehyd-Harnstoff-Harze, Stärke, Carboxymethylcellulose.

Die zu untersuchenden Proben stammen bevorzugt aus der papierverarbeitenden Industrie. Weitere Einsatzgebiete sind die Pigmentindustrie und die metallverarbeitende Industrie.

Erfindungsgemäß wird eine Probe der auf die mikrobielle Kontamination zu untersuchenden Suspensionen, Emulsionen oder Dispersionen genommen. Die Menge der entnommenen Probe beträgt im allgemeinen 0,5 - 20 ml, kann jedoch auch darunter oder darüber liegen. Im übrigen ist die Menge der entnommenen Probe für den Erfolg des erfindungsgemäßen Verfahrens nicht entscheidend.

Zur entnommenen Probe werden durch Mikroorganismen abbaubare organische Substanzen gegeben und miteinander vermischt. Der Anteil der zur Probe zugegebenen bioabbaubaren organischen Substanzen beträgt, pro ml Probe, 0,5 - 50 ml der bioabbaubaren Substanz.

Das Verhältnis von Probevolumen zum Volumen der bioabbaubaren Substanz ist abhängig von der Originalkonzentration der Probe und der Konzentration der bioabbaubaren Substanz in Lösung. Das Verhältnis wird so eingestellt, daß die Menge der bioabbaubaren Substanz so groß ist, daß eine Abtrennung der Mikroorganismen und der Mineralien, Füllstoffe, Pigmente, und/oder Fasermaterialien und wahlweise zusätzlich der Polymere in kolloidaler Form ermöglicht wird. Das jeweils zu ermittelnde optimale Verhältnis von Probevolumen zu Volumen der bioabbaubaren Substanz kann vom Fachmann durch Handversuche ermittelt werden.

Üblicherweise wird die Konzentration der Lösung, enthaltend die bioabbaubaren Substanz, so gewählt, dass das Verhältnis von Probelösung zur die bioabbaubaren Substanz enthaltende Lösung 1 : 0,1 bis 1 : 100 beträgt. Das optimale Verhältnis ist stark von der Konzentration und der Zusammensetzung der entsprechenden Probelösung abhängig. Pigment- und/oder Füllstoffsuspensionen mit einem Feststoffgehalt von > 65 Gew.% werden üblicherweise im Verhältnis 1 : 2 bis 1:10, bevorzugt 1:3, verdünnt. Bei einem Feststoffgehalt von < 65 Gew.% wird üblicherweise im Verhältnis 1 : 0,1 bis 1 : 1 verdünnt. In gewissen Fällen, speziell wenn eine sehr hohe Verkeimung erwartet wird, wird üblicherweise im Verhältnis 1 : 10 bis 1 : 100 verdünnt. Der gewählte Verdünnungsfaktor ist in der späteren Berechnung der Verkeimung zu berücksichtigen oder im Resultat speziell zu vermerken.

Zu den bioabbaubaren organischen Substanzen gehören insbesondere die Gruppe der Nährmedien, die für eine bestimmte, zu untersuchende Mikroorganismenspezies spezifisch sind. Nährmedien enthalten bevorzugt eine Kohlenstoff-, Stickstoff-, Phosphat- und/oder Schwefelquelle sowie wahlweise Mineralstoffe, Wuchsstoffe und/oder Vitamine. Weitere Stoffe können dem Nährmedium zugefügt werden, falls dies für das optimale Wachstum der Mikroorganismen erforderlich ist. Bevorzugte, erfindungsgemäß einsetzbare Nährmedien werden nachfolgend genauer beschrieben.

Nach Zugabe des Nährmediums können wahlweise ein oder mehrere Inkubationsschritte erfolgen, um die Anzahl der Mikroorganismen in der Probe zu erhöhen. Dies kann speziell für die spätere qualitative Analyse von Vorteil sein. In einer bevorzugten Ausführungsgestaltungsform der Erfindung findet diese Inkubation vor der Zentrifugation jedoch nicht statt, was zu einer deutlichen Zeitersparnis führt.

Um die Mikroorganismen von den Mineralien, Füllstoffen, Pigmenten und/oder Fasermaterialien abzutrennen, erfolgt nach Zugabe des Nährmediums und dem sich wahlweise anschließenden Inkubationsschritt ein Zentrifugationsschritt. Die Zentrifugation wird so durchgeführt, daß die überwiegende Anzahl der Mikroorganismen, d.h. über 50%, von den "Fremdstoffteilchen", also den Mineralien, Füllstoffen, Pigmenten, Polymeren und Fasermaterialien, abgetrennt werden. Die Zentrifugation muß so durchgeführt werden, daß sich die Mikroorganismen in der oberen Phase ansammeln, während die Fremdstoffteilchen sedimentieren. Zu diesem Zweck wird die Zentrifugation bevorzugt bei 100 bis 1500 g, bevorzugt 200 bis 1200 g und insbesondere bevorzugt 600 bis 1000 g, durchgeführt. Das Schwerefeld wird in Abhängigkeit von den abzutrennenden Mineralien, Füllstoffen, Pigmenten und Fasermaterialien bzw. in Abhängigkeit von den abzutrennenden Mikroorganismen eingestellt.

Die optimale Sedimentationszahl kann vom Fachmann aufgrund von Versuchen ermittelt werden. Die Zentrifugation selbst wird für einen Zeitraum von 1 bis 30 Minuten, bevorzugt 2 bis 15 Minuten und insbesondere bevorzugt 5 bis 10 Minuten, durchgeführt. Die optimale Zentrifugationszeit kann vom Fachmann durch Laborversuche ermittelt werden.

Als Mineralien und/oder Füllstoffe und/oder Pigmente werden bevorzugt verwendet: Elemente der zweiten und/oder dritten Hauptgruppe und/oder der vierten Hauptgruppe und/oder der vierten Nebengruppe des Periodensystems der Elemente, insbesondere Calcium und/oder Silicium und/oder Aluminium und/oder Titan und/oder Barium enthaltende Verbindungen und/oder organische Pigmente.

Bevorzugt eingesetzt werden als Mineralien, Füllstoffe und Pigmente Kaolin und/oder Aluminiumhydroxid und/oder Titanoxid und/oder Bariumsulfat und/oder Polystyrolhohlkugeln und/oder Formaldehydharze und/oder Calciumcarbonat enthaltende Mineralien und/oder Füllstoffe und/oder Pigmente, insbesondere natürliche Calciumcarbonate und/oder Marmor und/oder Kalk und/oder Dolomit und/oder Dolomit enthaltende Calciumcarbonate und/oder synthetisch hergestellte Calciumcarbonate, sogenannte präzipitierte Calciumcarbonate.

Der die Mikroorganismen enthaltende Überstand wird abgenommen und kann dann direkt einer quantitativen und/oder qualitativen Bestimmung der mikrobiellen Kontamination zugeführt werden. Bevorzugt schließen sich hier ein oder mehrere Inkubationsschritte an, um die Anzahl der Mikroorganismen im Überstand zu vermehren. Dieser Vermehrungsschritt erfolgt bei einer für die Mikroorganismen günstigen Inkubationstemperatur, die von der Art der zu vermehrenden Organismen abhängt. Bevorzugte Inkubationstemperaturen liegen im Bereich von 20 bis 37°C, weiterhin bevorzugt 28 bis 34°C und insbesondere bevorzugt 31,5 bis 32,5°C. Die jeweils notwendigen Inkubationstemperaturen sind dem Fachmann bekannt und können entweder aus Nachschlagewerken oder durch Versuche ermittelt werden.

Die Gesamtzeit der einzelnen Inkubationszeiten bei 30°C Inkubationstemperatur beträgt bei einer Verkeimung von weniger als 10⁵ Keimen/ml Probe bis zu 12 Stunden, bei einer Verkeimung von mehr als 10⁵ Keimen/ml Probe aber weniger als 10⁶ Keimen/g bis zu 6 Stunden, bei einer Suspension mit ursprünglich 60-80 Gew.-% Feststoffanteil mit einer Verkeimung von mehr als 10⁵ Keimen/ml (unter Verwendung von Tryptic Soy Broth Agar) bis zu 3 Stunden.

Die Summe der einzelnen Inkubationsschritte bei 30°C beträgt bei einer Verkeimung von weniger als 10⁵ Keimen/ml Probe 2 bis 6, bei einer Verkeimung von mehr als 10⁵ Keimen/ml Probe aber weniger als 10⁶ Keimen/g 2 bis 4, bei einer Suspension mit ursprünglich 60-80 Gew.-% Feststoffanteil mit einer Verkeimung von mehr als 10⁵ Keimen/ml (unter Verwendung von Tryptic Soy Broth Agar) 2 bis 3.

Die einzelnen Inkubationszeiten bei 30°C Inkubationstemperatur beträgt bei einer Verkeimung von weniger als 10⁵ Keimen/ml Probe 1 bis 12 Stunden, bei einer Verkeimung von mehr als 10⁵ Keimen/ml Probe aber weniger als 10⁶ Keimen/g 1 bis 6 Stunden, bei einer Suspension mit ursprünglich 60-80 Gew.-% Feststoffanteil mit einer Verkeimung von mehr als 10⁵ Keimen/ml (unter Verwendung von Tryptic Soy Broth Agar) 1 bis 2 Stunden.

Zur quantitativen bzw. qualitativen Bestimmung der so erhaltenen Mikroorganismen stehen verschiedene aus dem Stand der Technik bekannte Methoden zur Verfügung. Insbesondere bevorzugt wird erfindungsgemäß die Cellfacts^{R}-Analyse oder die ATP-Methode. Andere Verfahren stehen zur Verfügung und sind dem Fachmann bekannt. Die bevorzugten Verfahren werden in den nachfolgenden Beispielen näher beschrieben.

Nach Durchführen des erfindungsgemäßen Verfahrens ist eine qualitative und/oder quantitative Bestimmung der Mikroorganismen möglich. Unter qualitativer Bestimmung ist zunächst eine Grobunterscheidung zwischen den Gruppen Pilze, Hefen und Bakterien zu verstehen. Nach Eichung der Cellfacts^{R}-Analyse mit speziellen Mikroorganismen, beispielsweise speziellen Bakterien, ist auch eine weitere Spezifizierung in den einzelnen Unterarten möglich. Die qualitative Analyse über beispielsweise das Cellfacts^{R}-Verfahren findet aufgrund einer Differenzierung nach Grösse bzw. Volumen einer einzelnen Zelle statt.

Die Art der als Trennmittel eingesetzten Nährmedien hängt insbesondere von dem abzutrennenden und zu vermehrenden Mikroorganismus ab. Bevorzugt werden solche Nährmedien eingesetzt, die ein selektives Wachstum der zu bestimmenden Keime ermöglichen und das Wachstum anderer, nicht zu bestimmender Keime möglichst unterdrücken. Beispiele für erfindungsgemäß einsetzbare Nährmedien sind Tryptone Azolectin/Tween 20 (TAT) Broth Agar, Glukoselösung, Pepton/Casein, Nutrient Broth, bevorzugt Tryptic Soy Broth Agar. Die Zusammensetzung der Nährmedien ist im Anhang an diese Beschreibung angeführt.

Die Konzentration des Nährmediums für die Mikroorganismen liegt vorzugsweise bei 0,1 bis 10 Gew.-%, vorteilhafter bei 2 bis 5 Gew.-% und insbesondere vorteilhaft bei ca. 3 Gew.-%.

Durch das erfindungsgemäße Verfahren ist sowohl eine qualitative als auch eine quantitative Bestimmung der Bakterien, Pilze und Hefen möglich. In einer bevorzugten Ausführungsform erfolgt eine derartige Analyse in einem Schritt.

Nach der Abtrennung der Mikroorganismen von den Mineralien, Füllstoffen, Pigmenten und/oder Fasermaterialien, also den "Fremdstoffen", werden ein oder mehrere, bevorzugt zwei bis fünf Inkubationsschritte durchgeführt, um die Zahl der im Probenüberstand befindlichen Mikroorganismen zu vermehren.

Mehrmalige Inkubationen erfolgen in Intervallen, d.h. die Messung der Kontamination wird nach der Zeit tₒ₊ₓ je wiederholt. Hierdurch ist es möglich, die Inkubationszeit und die Inkubationsintervalle zu begrenzen. Zu dem Zeitpunkt, bei dem ein exponentielles Wachstum ersichtlich ist, wird zurück extrapoliert und auf die Zeit tₒ gerechnet. Ein hoher ursprünglicher Verkeimungsgrad wird zu einem früher feststellbaren und berechenbaren Wachstum führen, so daß auf nachfolgende Inkubationsintervalle verzichtet werden kann.

Die Inkubationszeit bei einer Verkeimung von weniger als 10⁵ Keimen/ml Probe beträgt bis zu 12 Stunden, bei einer Verkeimung von weniger als 10⁶ Keimen/ml bis zu 6 Stunden, bei einer Suspension mit ursprünglich 60-80 Gew.-% Feststoffanteil mit einer Verkeimung von mehr als 10⁵ Keimen/ml Probe bis zu 3 Stunden, wobei im letzteren Fall bevorzugt Tryptic Soy Broth Agar eingesetzt wird. Die in Abhängigkeit vom Kontaminationsgrad mit Mikroorganismen anzuwendende Inkubationszeit kann vom Fachmann durch einfache Handversuche ermittelt werden. Je geringer der ursprüngliche mikrobielle Kontaminationsgrad der Probe ist, desto länger müssen die Inkubationszeiten gewählt werden und umgekehrt. Der ursprüngliche Feststoffanteil der Suspension beeinflußt die Inkubationszeit nicht direkt.

Nachfolgend wird anhand von Vergleichsbeispielen und Ausführungsbeispielen die Erfindung näher erläutert. Weitere Abwandlungen der Erfindung können vom Fachmann aufgrund der vorliegenden Beschreibung in Verbindung mit den anliegenden Patentansprüchen sowie aufgrund des vorauszusetzenden Fachwissens durchgeführt werden. Die Erfindung ist nicht auf die vorliegenden Beispiele beschränkt.

### Keimzahlbestimmung mit dem Cellfacts^{R} Teilchenzähler

Die Mikrooganismen aus der Suspension und/oder Emulsion und/oder Dispersion werden erfindungsgemäß durch eine Meßpore mit definiertem Durchmesser mittels Vakuum gesaugt. An dieser Pore (Kapillare, 30µm) liegt eine Spannung (Volt) an. Da intakte Zellen in erster Näherung als Isolatoren betrachtet werden können, kommt es beim Durchtritt einer Zelle durch die Meßpore zu einer meßbaren Widerstandserhöhung, deren Größe vom Volumen der Zelle abhängt. Der Stromimpuls ist direkt proportional zum Volumen. Durch Inkubation der Proben können kleine Differenzen im Wachstum der Zellen gemessen werden. d.h. bei Zellteilung erhöht sich die Anzahl der Teilchen proportional. Da die Funktion des Zellwachstums exponentiell ist, kann nach dem Erkennen des Erreichens der exponentiellen Phase auf die Ausgangskonzentration zurück extrapoliert werden. Die Bestimmungszeit für CaC0₃-Talk- und Kaolinslurries sowie andere Stoffe (z.B. Kühlschmierstoffe, Siebwasser, kolloidal suspendierte Stärke etc.) liegt durch die erfindungsgemäße Präparation der industriellen Proben und unter Anwendung der obigen Methode bei nur 2 - 12 Stunden anstatt wie beim Stand der Technik bei 24 - 48h.

Dies erlaubt ein rechtzeitiges Eingreifen mit Konservierungsstoffen vor einer hohen Kontamination und/oder dem Verderb der Ware.

### Beispiele zum Stand der Technik

### 1. Beispiel, Calciumcarbonat Slurry

A) Keimzahlbestimmungen nach Schweizerisches Lebensmittelbuch, Kapitel 56, Abschnitt 7.01, Ausgabe 1985, Revision 1988, "Bestimmung von aeroben Bakterien und Keime" und nach dem Schweizerischen Lebensmittelbuch, Kapitel 56, Abschnitt 7.22, Ausgabe 1985, Revision 1988, "Bestimmung von Pilzen"

### Vorgehensweise

3 ml einer 77.5 Gew.-% wässerigen Aufschlämmung von natürlichem, gemahlenem Marmor aus Norwegen (90 Gew.-% der Teilchen < 2 µm, 65 Gew.-% der Teilchen < 1 µm), dispergiert mit 0.65 Gew.-% eines handelsüblichen Natriumpolyacrylats wurden nach der Methode "Bestimmung von aeroben mesophilen Keimen", Schweizerisches Lebensmittelbuch, Kapitel 56, Abschnitt 7.01, Ausgabe 1985, Revision 1988, analysiert.

### Resultat :

Die Keimzahlbestimmung ergab nach 48 Stunden Inkubationszeit: 3.0 x 10⁶ aerobe mesophile Keime / ml Suspension. Pilze wurden mit dem verwendeten Nährmedium für aerobe mesophile Keime nicht gefunden. Nach der im Schweizerisches Lebensmittelbuch, Kapitel 56, Abschnitt 7.22, Ausgabe 1985, Revision 1988, "Bestimmung von Pilzen" beschrieben Methode wurden < 10² Pilze/ml gefunden.

### B) Keimzahlbestimmung durch CellFacts Partikelmessgerät

### Vorgehensweise

3 ml der in Beispiel 1 A) verwendeten 77.5 Gew.-% wässerige Aufschlämmung von natürlichem, gemahlenem Marmor aus Norwegen (90 Gew.-% der Teilchen < 2 µm, 65 Gew.-% der Teilchen < 1 µm), dispergiert mit 0.65 Gew.-% eines handelsüblichen Natriumpolyacrylats wird in ein steriles Fläschchen pipetiert. Die Probe wird mit 3 ml 3 Gew.%iger TAT Broth Base / Tween 20 Nährlösung versetzt und mit dem CellFacts analysiert. Die Analyse erfolgt in der Weise, dass sofort (Zeit t₀ₕ) versucht wurde, die Anzahl Teilchen im Cellfacts Gerät zu messen. Da die Teilchenzahl viel zu hoch war, verlangte das Gerät eine Verdünnung der Probe. Nach einer Verdünnung von 1 : 10'000 wurde die Teilchenkonzentration vom Gerät akzeptiert, und es konnte der Blindwert (t₀ₕ) bestimmt. Nach 2 Std. (Zeit t₂ₕ), 4 Std. (Zeit t₄ₕ), nach 6 Std. (Zeit t₆ₕ), nach 12 Std. (Zeit t₁₂ₕ) Inkubationszeit wurde die Anzahl Teilchen im Cellfacts Gerät gemessen. Die Inkubationstemperatur betrug 30°C. Indem man die Anzahl Teilchen gegen den Durchmesser dieser Teilchen aufträgt, kann man nach bestimmten Inkubationszeiten der klaren Phase bestimmen, ob lebende Zellen vorhanden sind ("Peakerhöhung", y-Achse) und um welche Art von Mikroorganismen es sich handelt z.B. Bakterien, Hefen oder Pilze (mittlerer Teilchen ∅, x-Achse).

Nach 12 Stunden Inkubationszeit wird durch Extrapolation zurück auf die Zeit (ₜ₀ₕ) die ursprüngliche Verkeimung der Suspension ermittelt. Im Vergleich dazu dient das Resultat aus Beispiel 1 A) Keimzahlbestimmung nach dem Schweizerischen Lebensmittelbuch. ("Bestimmung von aeroben mesophilen Keimen", Schweizerisches Lebensmittelbuch, Kapitel 56, Abschnitt 7.01, Ausgabe 1985, Revision 1988)

### Resultat :

### Keimzahlbestimmung mit dem Cellfacts Teilchenzähler

### 1. Tryptic Soy Broth Agar

| Probe | Extraktionsmittel | Bestimmungszeit in Std. | aerobe mesophile Keime / ml Suspension *(Peakmax. bei Teilchen ∅ 1 - 2.5*µ*m)* |
|---|---|---|---|
| 1 | Tryptic Soy Broth Agar | 12 | < 10² |

| Probe | Extraktionsmittel | Bestimmungszeit in Std. | Pilze / ml Suspension (Peakmax. bei Teilchen ∅ 5 - 9µm) |
|---|---|---|---|
| 1 | Tryptic Soy Broth Agar | 12 | < 10² |

Aus Beispiel 1 A) und 1 B) geht klar hervor, dass die Bestimmung von Bakterien, Pilzen und Hefen in der obigen Suspension mit den Methoden des Standes der Technik einerseits mit dem CellFacts Gerät nicht möglich, respektive mit < 10² Keimen/ml ein völlig falsches Resultat liefert oder die Bestimmung nach der Methode des Schweizerischen Lebensmittelbuchs 48 Std. dauert.

Beim Cellfacts Gerät führte die notwendige Verdünnung der Probe vermutlich ebenfalls zu einer Verdünnung der anwesenden mikrobiologischen Keime unterhalb deren Nachweisgrenze.

Bei der Bestimmung nach dem Schweizerischen Lebensmittelbuch dauert die Analyse 48 Std.

### C) Keimzahlbestimmung durch ATP Messung

### Vorgehensweise

3 ml Probe wie in Beispiel 1 A), 77.5 Gew.-% wässerige Aufschlämmung von natürlichem, gemahlenem Marmor aus Norwegen (90 Gew.-% der Teilchen < 2 µm, 65 Gew.-% der Teilchen < 1 µm), dispergiert mit 0.65 Gew.-% eines handelsüblichen Natriumpolyacrylats werden in ein steriles Fläschchen pipettiert. Die Probe wird mit 3 ml 3 Gew.%iger TAT Broth Base / Tween 20 Nährlösung versetzt und mit dem BioOrbit Luminometer 1253 analysiert. Die Analyse erfolgt in der Weise, dass sofort (Zeit t₀ₕ) versucht wurde, die durch das ATP freigesetzte Lichtmenge im BioOrbit Luminometer 1253 zu messen. Ebenfalls wurde eine Probe nach einer Verdünnung von 1:10'000 gemessen. Es wurde so jeweils der Blindwert (t₀ₕ) bestimmt. Nach 4 Std. (Zeit t₄ₕ), nach 7 Std. (Zeit t₇ₕ) und nach 17 Std. (Zeit t₁₇ₕ) Inkubationszeit wurde wiederum die Lichtintensität im BioOrbit 1253 Luminometer gemessen. Die Inkubationstemperatur betrug 30°C. Indem man die Lichtintensität nach entsprechender Inkubationszeit gegen die Inkubationszeit aufträgt, kann man bestimmen, ob lebende Zellen vorhanden sind (Zunahme der Lichtintensität über die Zeit). Nach einer Inkubationszeit, bei welcher das Wachstum annäherungsweise exponentiell verläuft, kann durch Vergleich des Kurvenverlaufs der einzelnen Proben miteinander die Verkeimung in der Suspension vergleichend ermittelt werden. Es kann dadurch semiquantitativ zwischen "kein Wachstum", "schwaches Wachstum" und starkes Wachstum" unterschieden werden.

### Vorgehen beim Messen im Luminometer 1253

a) Zu Beginn werden von der, wie oben beschrieben vorbereiten Proben je 100 µl in eine Luminometer-Küvette pipettiert.
b) Dazu werden 100 µl ATP Releasing Reagent addiert, gut geschüttelt und für eine Minute stehen gelassen, damit ATP herausextrahiert werden kann.
c) Danach werden 500 µl AMR-Reagenz beigefügt und durch Schütteln vermischt.
d) Die Küvette kann nun in die Messzelle eingeführt werden und die Lichtemission abgelesen werden.

Literatur: Betriebsanleitung BioOrbit Luminometer Ver. 3.0, Mai '95 sowie die Application Note 20 der Firma BoiOrbit OY, FIN 20521 Turku, Finnland

| Resultat : | | |
|---|---|---|
| Inkubationszeit in Stunden | Probe Lichtintensität [RLU] am LCD Display | Probe 1:10'000 verdünnt, Lichtintensität [RLU] am LCD Display |
| 0 | 0.001 | 0.002 |
| 4 | 0.000 | 0.001 |
| 7 | 0.002 | 0.002 |
| 17 | 0.001 | 0.001 |

Bei diesem Verfahren aus dem Stand der Technik ist nach 17 Stunden kein Unterschied zwischen den einzelnen Inkubationszeiten ersichtlich, d.h. es muss angenommen werden, dass der Slurry nicht verkeimt ist. Da es sich jedoch um den gleichen Slurry wie in Beispiel 1 A (Methode nach Schweizerischem Lebensmittelbuch) handelt und die dort angewendete, anerkannte Methode ein Resultat von 3 x 10⁶ Keimen/ml ergibt, wird klar, dass es sich in diesem Beispiel 1C) um ein Fehlresultat handelt, was in der Praxis zu fatalen Folgen , wie Lebensmittelvergiftung oder Verderb von Gütern, führen kann.

Die Bestimmung von Bakterien, Pilzen und Hefen in der obigen Suspension ist mit dieser Methoden des Standes der Technik nicht möglich.

Vermutlich führte die hohe Teilchenkonzentration in der Originalkonzentration der Suspension zu vollständiger Lichtadsorption der durchs ATP freigesetzten Lichtmenge in der Suspension, und die Verdünnung der Probe führte ebenfalls zu einer Verdünnung der anwesenden mikrobiologischen Keime unterhalb deren Nachweisgrenze mit dieser Methode.

### Erfinderische Beispiele :

### 2 Beispiel, Calciumcarbonat Slurry, Analysengerät CellFacts Messgerät

### Vorgehensweise

3 ml einer 77.5 Gew.-% wässerigen Aufschlämmung von natürlichem, gemahlenem Marmor aus Norwegen (90 Gew.-% der Teilchen < 2 µm, 65 Gew.-% der Teilchen < 1 µm), dispergiert mit 0.65 Gew.-% eines handelsüblichen Natriumpolyacrylats werden je in ein separates, steriles Fläschchen pipetiert. Eine Probe wird mit 3 ml 3 Gew.%iger TAT Broth Base / Tween 20 Nährlösung versetzt und die zweite Probe mit 3 ml 3 Gew.%iger Tryptic Soy Broth Nährlösung. Anschliessend werden beide Proben 20 sec. gut geschüttelt und anschliessend zentrifugiert. Nach 10 min. Zentrifugation bei 800 g wird die obenstehende klare Phase isoliert und mit dem CellFacts analysiert. Die Analyse erfolgt in der Weise, dass sofort nach der Zentrifugation (Zeit t₀ₕ), nach 2 Std. (Zeit t₂ₕ), 4 Std. (Zeit t₄ₕ) und nach 6 Std. (Zeit t₆ₕ₎ Inkubationszeit die Anzahl Teilchen im Cellfacts Gerät gemessen wird. Die Inkubationstemperatur betrug 30°C. Indem man die Anzahl Teilchen gegen den Durchmesser dieser Teilchen aufträgt, kann man nach bestimmten Inkubationszeiten der klaren Phase bestimmen, ob lebende Zellen vorhanden sind (Peakerhöhung) und um welche Art von Mikroorganismen es sich handelt (Bakterien, Hefen oder Pilze). Nach einer Inkubationszeit, bei welcher das Wachstum exponentiell verläuft, wird durch Extrapolation zurück auf die Zeit t₀ₕ die ursprüngliche Verkeimung der Suspension ermittelt. Im Vergleich dazu wurde eine Keimzahlbestimmung nach dem Schweizerischen Lebensmittelbuch durchgeführt. ("Bestimmung von aeroben mesophilen Keimen", Schweizerisches Lebensmittelbuch, Kapitel 56, Abschnitt 7.01, Ausgabe 1985, Revision 1988)

### Resultate:

### Keimzahlbestimmung nach dem Schweizerischen Lebensmittelbuch

Die Keimzahlbestimmung ergab nach 48 Stunden : 3.0 x 10⁶ aerobe mesophile Keime / ml Suspension

Keimzahlbestimmung mit dem Cellfacts Teilchenzähler
1. TAT Broth Base / Tween 20 (Poly-oxyethylen-sorbitan-monolaurat)
2. Tryptic Soy Broth Agar

| Probe | Extraktionsmittel | Notwendige Bestimmungszeit in Std. | aerobe mesophile Keime / ml Suspension (Peakmax. bei Teilchen ∅ 1 - 2.5µm) |
|---|---|---|---|
| 1 | TAT Broth Base / Tween 20 | 6 | 1.25 x 10⁶ |
| 2 | Tryptic Soy Broth Agar | 4 | 3.14 x 10⁶ |

| Probe | Extraktionsmittel | Bestimmungszeit in Std. | Pilze / ml Suspension (Peakmax.. bei Teilchen ∅ 5 - 9µm) |
|---|---|---|---|
| 2. | Tryptic Soy Broth Agar | 12 | < 10² |

Die, nach dem erfindungsgemässen Verfahren analysierten Proben ergeben eine ausgezeichnete Übereinstimmung mit der Keimzahlbestimmung nach dem Schweizerischen Lebensmittelbuch, ermittelt nach 48 Stunden Inkubationszeit.
Beide Proben liegen gegenüber der Keimzahlbestimmung nach dem Schweizerischen Lebensmittelbuch sehr gut innerhalb des Vertrauensbereichs.

Unter Vetrauensbereich wird hier die Abweichung von +- 0.5 log₁₀ einzelner Keimzahlbestimmungen verstanden wie sie von der PHLS Central Public Health Laboratory, 61 Colindale Avenue, London NW9 5HT, UK, Distribution 070, Seite 6 u. 7 u. Appendix 1, Distribution Date 16. Nov. 1998, Report Date 30. Dez. 1998 erarbeitet wurde.

### 3) Beispiel Calciumcarbonat Slurry, Analysengerät BoiOrbit 1253 Luminometer

### Vorgehensweise

Jeweils 500 ml einer 71.5 Gew.-% wässerigen Aufschlämmung von natürlichem, gemahlenem Marmor (90 Gew.-% der Teilchen < 2 µm, 65 Gew.-% der Teilchen < 1 µm), dispergiert mit 0.5 Gew.-% eines handelsüblichen Natriumpolyacrylats, wurden in 1 Liter Glasflaschen abgefüllt. Die eine Probe wurde 3 Tage bei 5°C gelagert (Probe A), die andere wärend 3 Tagen bei 30°C (Probe B). Anschliessend wurden die Proben auf 20°C temperiert und jeweils 3 ml Probe in einem sterilen Fläschchen mit 3 ml des jeweiligen Extraktionsmittel gut vermischt. Nach 10 min. Zentrifugation bei 600 g wird die obenstehende klare Phase isoliert und mit dem BioOrbit 1253 Luminometer auf Lichtemission hin analysiert. Die Analyse erfolgte in der Weise, dass sofort nach der Zentrifugation (Zeit t₀ₕ), nach 4 Std. (Zeit t₄ₕ), nach 7 Std. (Zeit t₇ₕ), nach 17 Std. (Zeit t₁₇ₕ), Inkubationszeit die Lichtintensität im BioOrbit 1253 Luminometer gemessen wurde. Die Inkubationstemperatur betrug 30°C. Indem man die Lichtintensität nach entsprechender Inkubationszeit gegen die Inkubationszeit aufträgt, kann man in der klaren Phase bestimmen, ob lebende Zellen vorhanden sind (Zunahme der Lichtintensität über die Zeit). Nach einer Inkubationszeit, bei welcher das Wachstum annäherungsweise exponentiell verläuft, kann durch Vergleich des Kurvenverlaufs der einzelnen Proben miteinander, die Verkeimung in der Suspension vergleichend ermittelt werden. Es kann dadurch semiquantitativ zwischen "kein Wachstum", "schwaches Wachstum" und starkes Wachstum" unterschieden werden. Im Vergleich dazu wurde eine Keimzahlbestimmung nach dem Schweizerischen Lebensmittelbuch durchgeführt. ("Bestimmung von aeroben mesophilen Keimen", Schweizerisches Lebensmittelbuch, Kapitel 56, Abschnitt 7.01, Ausgabe 1985, Revision 1988)

### Verwendete Extraktionsmittel :

1. Stand der Technik,
   0.9 Gew.%ige NaCl Lösung in dest Wasser, ohne organische, als Nährstoff dienende Substanz.
2. Erfinderisch,
   3 Gew.%ige organische Nährlösung (Tryptic Soy Broth Agar)

### Keimzahlbestimmung nach dem Schweizerischen Lebensmittelbuch

Probe A : 5 x 10⁴ aerobe mesophile Keime/ g Suspension nach 48 Stunden
Probe B : 7 x 10⁷ aerobe mesophile Keime/ g Suspension nach 48 Stunden

### Resultat :

### Beurteilung der Verkeimung mit dem BioOrbit Luminometer

| Stand der Technik, 0.9 Gew.%ige NaCl - Lösung in dest Wasser | | |
|---|---|---|
| Inkubationszeit in Stunden | Probe 1A, Lichtintensität [RLU] am LCD Display | Probe 1B, Lichtintensität [RLU] am LCD Display |
| 0 | 0.001 | 0.001 |
| 4 | 0.002 | 0.003 |
| 7 | 0.002 | 0.004 |
| 17 | 0.004 | 0.007 |

Beim Verfahren vom Stand der Technik ist nach 17 Stunden kein eindeutiger Unterschied zwischen der schwach und stark verkeimten Suspension möglich.

| Erfinderisch, 3 Gew.%ige organische Nährlösung (Tryptic Soy Broth Agar) | | |
|---|---|---|
| Inkubationszeit in Stunden | Probe 2A, Lichtintensität [RLU] am LCD Display | Probe 2B, Lichtintensität [RLU] am LCD Display |
| 0 | 0.001 | 0.002 |
| 4 | 0.004 | 0.006 |
| 7 | 0.007 | 0.045 |
| 17 | 0.050 | 0.240 |

Bei Verwendung des erfindungsgemässen neuen Verfahren ist eine Unterscheidung zwischen "schwach" und "stark verkeimter" Suspension bereits nach 7 Stunden möglich.
Bereits nach 17 Stunden ist ebenfalls eine "schwache Verkeimung" signifikant zu erkennen.

In der Literatur und: Betriebsanleitung vom BioOrbit Luminometer Ver. 3.0, Mai '95 sowie in der "Application Note 20" der Firma BoiOrbit OY, FIN 20521 Turku, Finnland werden auch Berechnungsmodelle gezeigt wie von den [RLU]-Werten auf die Boimasse gerechnet werden kann. Weiterhin besteht die Möglichkeit durch "Eichung" mit Suspensionen mit bekanntem Verkeimungsgrad auf eine Keimzahl / ml zu schliessen.

### 4. Beispiel, US Clay Slurry

### Vorgehensweise

3 ml einer 72.8 Gew.-% wässerigen Aufschlämmung von natürlichem, gemahlenem US Clay aus Georgia, USA (95 Gew.-% der Teilchen < 2 µm, 78 Gew.-% der Teilchen < 1 µm), dispergiert mit 0.35 Gew.-% eines handelsüblichen Natriumpolyacrylats werden je in ein separates, steriles Fläschchen pipetiert. Eine Probe wird mit 3 ml 3 Gew.%iger TAT Broth Base / Tween 20 Nährlösung versetzt und die zweite Probe mit 3 ml 3 Gew.%iger Tryptic Soy Broth Nährlösung. Anschliessend werden beide Proben 20 sec. gut geschüttelt und anschliessend zentrifugiert. Nach 10 min. Zentrifugation bei 800 g wird die obenstehende klare Phase isoliert und mit dem Cellfacts analysiert. Die Analyse erfolgt in der Weise, dass sofort nach der Zentrifugation (Zeit t₀ₕ), nach 2 Std. (Zeit t₂ₕ), 4 Std. (Zeit t₄ₕ), nach 6 Std. (Zeit t₆ₕ), nach 12 Std. (Zeit t₁₂ₕ), Inkubationszeit die Anzahl Teilchen im Cellfacts Gerät gemessen werden. Die Inkubationstemperatur betrug 30°C. Indem man die Anzahl Teilchen gegen den Durchmesser dieser Teilchen aufträgt, kann man nach bestimmten Inkubationszeiten der klaren Phase bestimmen, ob lebende Zellen vorhanden sind (Peakerhöhung) und um welche Art von Mikroorganismen es sich handelt (Bakterien, Hefen oder Pilze). Nach einer Inkubationszeit, bei welcher das Wachstum exponentiell verläuft, wird durch Extrapolation zurück auf die Zeit t₀ₕ die ursprüngliche Verkeimung der Suspension ermittelt. Im Vergleich dazu wurde eine Keimzahlbestimmung nach dem Schweizerischen Lebensmittelbuch durchgeführt. ("Bestimmung von aeroben mesophilen Keimen" Schweizerisches Lebensmittelbuch, Kapitel 56, Abschnitt 7.01, Ausgabe 1985, Revision 1988)

### Keimzahlbestimmung nach dem Schweizerischen Lebensmittelbuch

Die Keimzahlbestimmung ergab nach 48 Stunden : 5.0 x 10⁵ aerobe mesophile Keime / ml Suspension.

### Keimzahlbestimmung mit dem Cellfacts Teilchenzähler

1. TAT Broth Base / Tween 20 (Poly-oxyethylen-sorbitan-monolaurat)
2. Tryptic Soy Broth Agar

| Probe | Extraktionsmittel | Notwendige Bestimmungszeit in Std. | aerobe mesophile Keime / ml Suspension (Peakmax. bei Teilchen ∅ 1 - 2.5µm) |
|---|---|---|---|
| 1 | TAT Broth Base / Tween 20 | 12 | 1.12 x 10⁵ |
| 2 | Tryptic Soy Broth Agar | 6 | 5.21 x 10⁵ |

| Probe | Extraktionsmittel | Bestimmungszeit in Std. | Pilze / ml Suspension (Peakmax. bei Teilchen ∅ 5 - 9µm) |
|---|---|---|---|
| 2 | Tryptic Soy Broth Agar | 12 | ca. 10³ |

Die nach dem erfindungsgemässen Verfahren analysierten Proben ergeben eine ausgezeichnete Übereinstimmung mit der Keimzahlbestimmung nach dem Schweizerischen Lebensmittelbuch, ermittelt nach 48 Stunden Inkubationszeit.
Beide Proben liegen gegenüber der Keimzahlbestimmung nach dem Schweizerischen Lebensmittelbuch sehr gut innerhalb des Vertrauensbereichs.

Unter Vetrauensbereich wird hier die Abweichung von +- 0.5 log₁₀ einzelner Keimzahlbestimmungen verstanden wie sie von der PHLS Central Public Health Laboratory, 61 Colindale Avenue, London NW9 5HT, UK, Distribution 070, Seite 6 u. 7 u. Appendix 1, Distribution Date 16. Nov. 1998, Report Date 30. Dez. 1998 erarbeitet wurde.

### 5. Beispiel, Siebwasser einer Papiermaschine

### Vorgehensweise

3 ml einer 0.5 Gew.-%igen wässerigen Aufschlämmung, enthaltend ca. 0.2 Gew.% natürlichen, gemahlenen Marmor aus Norwegen (60 Gew.-% der Teilchen < 2 µm, 35 Gew.-% der Teilchen < 1 µm), dispergiert mit 0.15 Gew.-% eines handelsüblichen Natriumpolyacrylats und ca. 0.3 Gew.% Cellulosefasern /Sulfit/Sulfat Zellstoff), wie sie in Papierfabriken zur Herstellung von Papier eingesetzt werden und ca. 0.05 Gew.% eines handelsüblichen Polyacrylamids, werden in ein steriles Fläschchen pipettiert. Die Probe wird mit 3 ml 3 Gew.%iger Tryptic Soy Broth Nährlösung versetzt. Anschliessend wird die Proben 20 sec. gut geschüttelt und anschliessend zentrifugiert. Nach 10 min. Zentrifugation bei 1000 g wird die obenstehende klare Phase isoliert und mit dem Cellfacts analysiert. Die Analyse erfolgt in der Weise dass sofort nach der Zentrifugation (Zeit t₀ₕ), nach 1 Std. (Zeit t₁ₕ), 2 Std. (Zeit t₂ₕ), Inkubationszeit die Anzahl Teilchen im Cellfacts Gerät gemessen werden. Die Inkubationstemperatur betrug 30°C. Indem man die Anzahl Teilchen gegen den Durchmesser dieser Teilchen aufträgt, kann man nach bestimmten Inkubationszeiten der klaren Phase bestimmen, ob lebende Zellen vorhanden sind (Peakerhöhung) und um welche Art von Mikroorganismen es sich handelt (Bakterien, Hefen oder Pilze). Nach einer Inkubationszeit, bei welcher das Wachstum exponentiell verläuft, wird durch Extrapolation zurück auf die Zeit t₀ₕ die ursprüngliche Verkeimung der Suspension ermittelt. Im Vergleich dazu wurde eine Keimzahlbestimmung nach dem Schweizerischen Lebensmittelbuch durchgeführt. ("Bestimmung von aeroben mesophilen Keimen", Schweizerisches Lebensmittelbuch, Kapitel 56, Abschnitt 7.01, Ausgabe 1985, Revision 1988)

### Keimzahlbestimmung nach dem Schweizerischen Lebensmittelbuch

Die Keimzahlbestimmung ergab nach 48 Stunden : 3.0 x 10⁷ aerobe mesophile Keime / ml Suspension.

### Keimzahlbestimmung mit dem Cellfacts Teilchenzähler

### 1. Tryptic Soy Broth Agar

| Probe | Extraktionsmittel | Notwendige Bestimmungszeit in Std. | aerobe mesophile Keime / ml Suspension (Peakmax. bei Teilchen ∅ 1 - 2.5µm) |
|---|---|---|---|
| 1 | Tryptic Soy Broth Agar | 2 | 3.45 x 10⁷ |

| Probe | Extraktionsmittel | Bestimmungszeit in Std. | Pilze / ml Suspension (Peakmax. bei Teilchen ∅ 5 - 9µm) |
|---|---|---|---|
| 1 | Tryptic Soy Broth Agar | 6 | ca. 10⁴ |

Die nach dem erfindungsgemässen Verfahren analysierten Proben ergeben eine ausgezeichnete Übereinstimmung mit der Keimzahlbestimmung nach dem Schweizerischen Lebensmittelbuch, ermittelt nach 48 Stunden Inkubationszeit.
Die Probe liegt gegenüber der Keimzahlbestimmung nach dem Schweizerischen Lebensmittelbuch sehr gut innerhalb des Vertrauensbereichs.

Unter Vetrauensbereich wird hier die Abweichung von +- 0.5 log₁₀ einzelner Keimzahlbestimmungen verstanden wie sie von der PHLS Central Public Health Laboratory, 61 Colindale Avenue, London NW9 5HT, UK, Distribution 070, Seite 6 u. 7 u.

Appendix 1, Distribution Date 16. Nov. 1998, Report Date 30. Dez. 1998 erarbeitet wurde.

### 6. Beispiel, kolloidale Stärkesuspension

### Vorgehensweise

3 ml einer 10 Gew.-%ige wässerige, kolloidale Suspension von Maisstärke, wie sie in Papierfabriken im Eisatz ist, wird in ein steriles Fläschchen pipetiert. Die Probe wird mit 3 ml 3 Gew.%iger Tryptic Soy Broth Nährlösung versetzt. Anschliessend wird die Proben 20 sec. gut geschüttelt und anschliessend zentrifugiert. Nach 10 min. Zentrifugation bei 800 g wird die obenstehende klare Phase isoliert und mit dem Cellfacts analysiert. Diese Analyse erfolgt in der Weise, daß sofort nach der Zentrifugation (Zeit t₀ₕ) nach 1 Std. (Zeit t₁ₕ), 2 Std. (Zeit t₂ₕ), Inkubationszeit die Anzahl der Teilchen im Cellfacts Gerät gemessen werden. Die Inkubationstemperatur betrug 30°C. Indem man die Anzahl Teilchen gegen den Durchmesser dieser Teilchen aufträgt, kann man nach bestimmten Inkubationszeiten der klaren Phase bestimmen, ob lebende Zellen vorhanden sind (Peakerhöhung) und um welche Art von Mikroorganismen es sich handelt (Bakterien, Hefen oder Pilze). Nach einer Inkubationszeit, bei welcher das Wachstum exponentiell verläuft, wird durch Extrapolation zurück auf die Zeit t₀ₕ die ursprüngliche Verkeimung der Suspension ermittelt. Im Vergleich dazu wurde eine Keimzahlbestimmung nach dem Schweizerischen Lebensmittelbuch durchgeführt. ("Bestimmung von aeroben mesophilen Keimen", Schweizerisches Lebensmittelbuch, Kapitel 56, Abschnitt 7.01, Ausgabe 1985, Revision 1988)

### Keimzahlbestimmung nach dem Schweizerischen Lebensmittelbuch

Die Keimzahlbestimmung ergab nach 48 Stunden : 4.0 x 10⁶ aerobe mesophile Keime / ml Suspension

### Keimzahlbestimmung mit dem Cellfacts Teilchenzähler

### 1. Tryptic Soy Broth Agar

| Probe | Extraktionsmittel | Notwendige Bestimmungszeit in Std. | aerobe mesophile Keime / ml Suspension (Peakmax. bei Teilchen ∅ 1 - 2.5µm) |
|---|---|---|---|
| 1 | Tryptic Soy Broth Agar | 2 | 4.22 x 10⁶ |

| Probe | Extraktionsmittel | Bestimmungszeit in Std. | Pilze / ml Suspension (Peakmax. bei Teilchen ∅ 5 - 9µm) |
|---|---|---|---|
| 1 | Tryptic Soy Broth Agar | 12 | < 10² |

Die, nach dem erfindungsgemässen Verfahren analysierte Probe ergibt eine ausgezeichnete Übereinstimmung mit der Keimzahlbestimmung nach dem Schweizerischen Lebensmittelbuch, ermittelt nach 48 Stunden Inkubationszeit.
Die Probe liegt gegenüber der Keimzahlbestimmung nach dem Schweizerischen Lebensmittelbuch sehr gut innerhalb des Vertrauensbereichs.

Unter Vetrauensbereich wird hier die Abweichung von +- 0.5 log₁₀ einzelner Keimzahlbestimmungen verstanden wie sie von der PHLS Central Public Health Laboratory, 61 Colindale Avenue, London NW9 5HT, UK, Distribution 070, Seite 6 u. 7 u. Appendix 1, Distribution Date 16. Nov. 1998, Report Date 30. Dez. 1998 erarbeitet wurde.

### 7. Beispiel

### Vorgehensweise

3 ml einer 71.5 Gew.-% wässerige Aufschlämmung von natürlichem, gemahlenem Marmor (90 Gew.-% der Teilchen < 2 µm, 65 Gew.-% der Teilchen < 1 µm), dispergiert mit 0.5 Gew.-% eines handelsüblichen Natriumpolyacrylats werden in einem sterilen Fläschchen mit 3 ml des jeweiligen Extraktionsmittel gut vermischt. Nach 10 min. Zentrifugation bei 800 g wird die obenstehende klare Phase isoliert und mit dem Cellfacts analysiert. Die Analyse erfolgt in der Weise dass sofort nach der Zentrifugation (Zeit t₀ₕ), nach 2 Std. (Zeit t₂ₕ), 4 Std. (Zeit t₄ₕ), nach 7 Std. (Zeit t₇ₕ), nach 17 Std. (Zeit t₁₇ₕ), und nach 24 Std. (Zeit t₂₄ₕ), Inkubationszeit die Anzahl Teilchen im Cellfacts Gerät gemessen werden. Die Inkubationstemperatur betrug 30°C. Indem man die Anzahl Teilchen gegen den Durchmesser dieser Teilchen aufträgt, kann man nach bestimmten Inkubationszeiten der klaren Phase bestimmen, ob lebende Zellen vorhanden sind (Peakerhöhung) und um welche Art von Mikroorganismen es sich handelt (Bakterien, Hefen oder Pilze). Nach einer Inkubationszeit, bei welcher das Wachstum exponentiell verlief, wurde durch Extrapolation zurück auf die Zeit t₀ₕ die ursprüngliche Verkeimung der Suspension ermittelt. Im Vergleich dazu wurde eine Keimzahlbestimmung nach dem Schweizerischen Lebensmittelbuch durchgeführt. ("Bestimmung von aeroben mesophilen Keimen", Schweizerisches Lebensmittelbuch, Kapitel 56, Abschnitt 7.01, Ausgabe 1985, Revision 1988)

### Verwendete Extraktionsmittel :

### Stand der Technik

1. dest. Wasser
2. 0.9 Gew.%ige NaCl Lösung in dest Wasser, anorganisches Salz
3. 0.8 Gew.%ige Lösung von Poly-oxyethylen-sorbitan-monolaurat (2 mol poly-oxyethylen), organisches Netzmittel

### Beispiel zur Erfindung

4. 3 Gew.%ige organische Nährlösung (Tryptic Soy Broth Agar)

### Keimzahlbestimmung nach dem Schweizerischen Lebensmittelbuch

Die Keimzahlbestimmung ergab nach 48 Stunden : 5.0 x 10⁴ aerobe mesophile Keime / ml Suspension

### Keimzahlbestimmung mit dem CellFacts Teilchenzähler

| Probe | Extraktionsmittel | Bestimmungszeit in Std. | aerobe mesophile Keime / ml Suspension (Peakmax. bei Teilchen ∅ 1 - 2.5µm) |
|---|---|---|---|
| 1 | dest. Wasser | 17 | 2.0 x 10³ |
| 2 | NaCl - Lösung | 18 | 1.6 x 10³ |
| 3 | Poly-oxyethylensorbitan-monolaurat | 24 | > 10² |
| | | | |
| 4 | organische Nährlösung | 7 | 5.6 x 10⁴ |

Aus den Proben 1 - 3 geht klar hervor, dass einerseits die notwendige Inkubationszeit sehr lang ist bis ein Resultat errechnet werden kann und das Resultat zudem falsch ist. (Vergleich : Keimzahlbestimmung nach dem Schweizerische Lebensmittelbuch)

Bei den Proben 1 - 3 liegt die Abweichung gegenüber der Keimzahlbestimmung nach dem Schweizerischen Lebensmittelbuch weit ausserhalb des Vertrauensbereichs.

Die Proben 1 und 2 enthalten keine organische, als Nährstoff wirkende Substanz. Die Probe 3 enthält eine organische Substanz die nicht als Nährstoff sondern als Hemmstoff dient. Bereits in Beispiel 4-1 war bei Einsatz von Tween 20 gegenüber Beispiel 4-2 eine gewisse Hemmung durch Tween 20 zu erkennen. Die erfindungsgemäss eingesetzte organische, als Nährlösung dienende Substanz konnte jedoch ein Fehlresultat in Beispiel 4-2 verhindern. In Beispiel 7-3 jedoch liegt gegenüber dem Beispiel 7-4 ein fatales Fehlresultat vor!

Die nach dem erfindungsgemässen Verfahren analysierte Probe 4 ergibt eine ausgezeichnete Übereinstimmung mit der Keimzahlbestimmung nach dem Schweizerischen Lebensmittelbuch.

Bei der Probe 4 liegt die Abweichung gegenüber der Keimzahlbestimmung nach dem Schweizerischen Lebensmittelbuch sehr gut innerhalb des Vertrauensbereichs.

Unter Vertrauensbereich wird hier die Abweichung von +- 0.5 log₁₀ einzelner Keimzahlbestimmungen verstanden wie sie von der PHLS Central Public Health Laboratory, 61 Colindale Avenue, London NW9 5HT, UK, Distribution 070, Seite 6 u. 7 u. Appendix 1, Distribution Date 16. Nov. 1998, Report Date 30. Dez. 1998 erarbeitet wurde.

| **Zusammensetzung bevorzugter Nährmedien** | |
|---|---|
| Tryptic Soy Broth | |
| Bacto-Trypton | 17,0 g/l |
| Bacto-Soyton | 3,0 g/l |
| Bacto-Dextrose | 2,5 g/l |
| Natriumchlorid | 5,0 g/l |
| Dicaliumphosphat | 2,5 g/l |

| TAT-Broth Base | |
|---|---|
| Bacto-Trypton | 20,0 g/l |
| Azolectin | 5,0 g/l |

| Nutrient Broth | |
|---|---|
| Bacto-Beef-Extrakt | 3,0 g/l |
| Bacto-Peptin | 5,0 g/l |

| Peptin aus Casein, tryptischer Verdau | |
|---|---|
| Peptin aus Casein | 1,0 g/l |
| Natriumchlorid | 1,8 g/l |

| Plate Count Agar | |
|---|---|
| Bacto-Trypton | 5,0 g/l |
| Hefeextrakt | 2,5 g/l |
| Bacto-Dextrose | 1,0 g/l |
| Agar Nr. 1 | 9,0 g/l |

| Ethylenglycol | |
|---|---|
| Ethylenglycol | 1,0 g/l |
| Azolectin | 5,0 g/l |
| Natriumchlorid | 1,8 g/l |

| Glycerin | |
|---|---|
| Glycerin | 1,0 g/l |
| Azolectin | 5,0 g/l |
| Natriumchlorid | 1,8 g/l |

## Patentansprüche

1. Verfahren zur quantitativen und/oder qualitativen Bestimmung der mikrobiellen Kontamination von Mineralien und/oder Pigmente und/oder Füllstoffe und/oder Fasermaterialien enthaltenden Suspensionen, Emulsionen oder Dispersionen,
**dadurch gekennzeichnet, daß**
a) eine Probe der Suspensionen, Emulsionen oder Dispersionen mit einer oder mehreren durch Mikroorganismen abbaubaren, als Trennmittel zwischen den Mikroorganismen und den Mineralien, Pigmenten, Füllstoffen und/oder Fasermaterialien wirkenden, organischen Substanzen vermischt wird, wobei die Menge an bioabbaubarer Substanz so gewählt wird, daß eine Abtrennung der Mikroorganismen und der Mineralien, Füllstoffe, Pigmente und/oder Fasermaterialien ermöglicht wird, und wahlweise zur Erhöhung der Anzahl der Mikroorganismen inkubiert wird,
b) die so erhaltene Mischung zentrifugiert wird, um die Mineralien und/oder Füllstoffe und/oder Pigmente und/oder Fasermaterialien mehrheitlich von den Mikroorganismen abzutrennen, wobei sich die Mikroorganismen in der oberen Phase befinden, und
c) der durch die Zentrifugation erhaltene wässrige Überstand bevorzugt einer Inkubation oder mehreren Inkubationen unterworfen wird, um die Anzahl der Mikroorganismen in diesem Überstand zu vermehren, und
d) die Anzahl und/oder Größe und/oder Art der Mikroorganismen im Überstand bestimmt wird.

2. Verfahren nach Anspruch 1,
**dadurch gekennzeichnet, daß** es sich bei der mikrobiellen Kontamination um Bakterien und/oder Pilze und/oder Hefen handelt.

3. Verfahren nach einem der Ansprüche 1-2,
**dadurch gekennzeichnet, daß**
die durch Mikroorganismen abbaubare organische Substanz in Form eines Nährmediums für Mikroorganismen zugesetzt wird.

4. Verfahren nach Anspruch 3,
**dadurch gekennzeichnet, daß**
das Nährmedium eine Kohlenstoff-, Stickstoff-, Phosphat- und/oder Schwefelquelle sowie wahlweise Mineralstoffe, Wuchsstoffe und/oder Vitamine enthält.

5. Verfahren nach einem oder mehreren der vorhergehenden Ansprüche,
**dadurch gekennzeichnet, daß**
die Zentrifugation bei 100-1.500 g, bevorzugt 200-1.200 g, besonders bevorzugt 600-1.000 g erfolgt.

6. Verfahren nach einem oder mehreren der vorhergehenden Ansprüche,
**dadurch gekennzeichnet, daß**
die Inkubationstemperatur 20-37°C, bevorzugt 28-34°C und am vorteilhaftesten 31,6-32,5°C beträgt.

7. Verfahren nach einem oder mehreren der vorhergehenden Ansprüche,
**dadurch gekennzeichnet, daß**
solche Nährmedien eingesetzt werden, die selektiv das Wachstum der zu bestimmenden Keime ermöglichen.

8. Verfahren nach einem oder mehreren der vorhergehenden Ansprüche,
**dadurch gekennzeichnet, daß**
als Nährmedium Tryptone Azolectin/Tween 20 (TAT) Broth Agar und/oder Glukoselösung und/oder Pepton/Casein und/oder Nutrient Broth, bevorzugt Tryptic Soy Broth Agar, verwendet wird.

9. Verfahren nach einem oder mehreren der vorhergehenden Ansprüche,
**dadurch gekennzeichnet, daß**
die Konzentration des Nährmediums für Mikroorganismen vorzugsweise 0,1-10 Gew.-%, vorteilhafter 2-5 Gew.-%, am vorteilhaftesten 3 Gew.-% beträgt.

10. Verfahren nach einem oder mehreren der vorhergehenden Ansprüche,
**dadurch gekennzeichnet, daß**
die Suspension, Emulsion oder Dispersion weiterhin zusätzlich Polymere in kolloidaler Form enthält.

11. Verfahren nach einem oder mehreren der vorhergehenden Ansprüche,
**dadurch gekennzeichnet, daß**
die qualitative und/oder quantitative Bestimmung von Bakterien und/oder Pilzen und/oder Hefen in einer Analyse nebeneinander erfolgt.

12. Verfahren nach einem oder mehreren der vorhergehenden Ansprüche,
**dadurch gekennzeichnet, daß**
der Zentrifugationsschritt für einen Zeitraum von 1 - 30 Min., bevorzugt 2 - 15 Min., besonders bevorzugt 10 Min. erfolgt.

13. Verfahren nach einem oder mehreren der vorhergehenden Ansprüche,
**dadurch gekennzeichnet, daß**
die durch die Zentrifugation erhaltene, Mikroorganismen aufweisende Phase durch ein Teilchengrößenanalyseverfahren auf die Menge der Mikroorganismen analysiert wird.

14. Verfahren nach Anspruch 13,
**dadurch gekennzeichnet, daß**
die Größe und Anzahl der Mikroorganismen analysiert werden, indem die Mikroorganismenprobe mit Hilfe eines Vakuums durch eine Meßpore mit definierter Länge und Durchmesser gesaugt wird, wobei zwischen Ein- und Ausgang der Pore eine Spannung anliegt, und bei Durchtritt von Mikroorganismen durch die Pore ein Stromimpuls gemessen wird, der direkt proportional zum Volumen des Mikroorganismus ist und die Zahl der Stromimpulse proportional zur Anzahl der Mikroorganismen ist.

15. Verfahren nach einem oder mehreren der vorhergehenden Ansprüche,
**dadurch gekennzeichnet, daß**
die durch die Zentrifugation erhaltene, Mikroorganismen aufweisende Phase durch Bestimmung des durch die Mikroorganismen produzierten ATP's auf die Menge der Mikroorganismen analysiert wird.

16. Verfahren nach einem oder mehreren der vorhergehenden Ansprüche,
**dadurch gekennzeichnet, daß**
die mikrobielle Kontamination von Dispersionen, Emulsionen und Suspensionen in der metallverarbeitenden Industrie, in der Pigment- und Papierindustrie sowie von Kreislaufwässern der Papierindustrie untersucht wird.

## Claims

1. Process for the quantitative and/or qualitative determination of the microbial contamination of suspensions, emulsions or dispersions containing minerals and/or pigments and/or fillers and/or fiber materials **characterized in that**
a) a sample of the suspensions, emulsions or dispersions, is mixed with one or more organic substances which are degradable by microorganisms and which are effective as separating agents between the microorganisms and the minerals, pigments, fillers and/or fiber materials, wherein the amount of bio-degradable substance is selected such that a separation of the microorganisms from the minerals, fillers, pigments and/or fiber materials is rendered possible, and wherein optionally an incubation is performed to increase the number of the microorganisms,
b) the mixture thus obtained is centrifuged in order to separate the majority of the minerals and/or fillers and/or pigments and/or fiber materials from the microorganisms, whereas the microorganisms are in the upper phase, and
c) the aqueous supernatant obtained by the centrifugation is preferably subjected to one incubation or several incubations in order to increase the number of microorganisms in said supernatant, and
d) the number and/or size and/or type of microorganisms in this supernatant is determined.

2. Process according to claim 1 **characterized in that** said microbial contamination are bacteria and/or fungi and/or yeasts.

3. Process according to any of claims 1 to 2 **characterized in that** said organic substance which is degradable by microorganisms is added in the form of a nutrient medium for microorganisms.

4. Process according to claim 3 **characterized in that** said nutrient medium contains a source of carbon, nitrogen, phosphate and/or sulfur as well as optionally minerals, growth substances and/or vitamins.

5. Process according to one or more of the preceding claims **characterized in that** the centrifugation is performed at 100-1,500 g, preferably 200-1,200 g, particularly preferred 600-1,000 g.

6. Process according to one or more of the preceding claims **characterized in that** the incubation temperature is 20-37°C, preferably 28-34°C and most advantageously 31.5-32.5°C.

7. Process according to one or more of the preceding claims **characterized in that** such nutrient media are used which enable the selective growth of the germs to be determined.

8. Process according to one or more of the preceding claims **characterized in that** as the nutrient medium tryptone azolectin/Tween 20 (TAT) broth agar and/or glucose solution and/or peptone/casein and/or nutrient broth, preferably tryptic soy broth agar, is used.

9. Process according to one or more of the preceding claims **characterized in that** the concentration of the nutrient medium for microorganisms is preferably 0.1-10 % by wt., more advantageously 2-5 % by wt. and most advantageously 3 % by wt.

10. Process according to one or more of the preceding claims **characterized in that** the suspension, emulsion or dispersion further contains polymers in a colloidal form.

11. Process according to one or more of the preceding claims **characterized in that** the qualitative and/or quantitative determination of bacteria and/or fungi and/or yeasts is performed simultaneously in one analysis.

12. Process according to one or more of the preceding claims **characterized in that** the centrifugation step is performed for a time of 1-30 min, preferably 2-15 min, particularly preferred 10 min.

13. Process according to one or more of the preceding claims **characterized in that** the phase obtained by centrifugation containing microorganisms is analyzed for the amount of microorganisms by means of a particle size analysis method.

14. Process according to claim 13 **characterized in that** the size and number of microorganisms is analyzed by aspirating the microorganism sample by means of a vacuum through a measurement pore having a defined length and diameter wherein a voltage is applied at the pore inlet and exit and a current pulse is measured upon passage of microorganisms through the pore which is directly correlated to the volume of the microorganism, and the number of current pulses is correlated to the number of microorganisms.

15. Process according to one or more of the preceding claims **characterized in that** the phase obtained by centrifugation containing microorganisms is analyzed for the amount of microorganisms by determination of the ATP produced by the microorganisms.

16. Process according to one or more of the preceding claims **characterized in that** the microbial contamination of dispersions, emulsions and suspensions in the metal industry, in the pigment and paper industry as well as in paper industry white waters is analysed.

## Revendications

1. Procédé de détermination quantitative et/ou qualitative de contamination microbienne de suspensions, d'émulsions ou de dispersion contenant des minéraux et/ou des pigments et/ou des charges et/ou des matériaux fibreux, **caractérisé en ce que**
a) un échantillon de suspensions, d'émulsions ou de dispersions est mélangé avec une ou plusieurs substances organiques agissant comme élément de séparation entre les microorganismes et les minéraux, pigments, charges et/ou matériaux fibreux dégradables par des microorganismes, la quantité de substance biodégradable étant choisie de telle sorte qu'une séparation des microorganismes et des minéraux, charges, pigments et/ou matériaux fibreux soit possible, et si besoin est incubé pour augmenter le nombre de microorganismes,
b) le mélange ainsi obtenu est centrifugé pour séparer les minéraux et/ou les charges et/ou les pigments et/ou les matériaux fibreux principalement des microorganismes, les microorganismes se trouvant dans la phase supérieure et,
c) le résidu aqueux obtenu après centrifugation est soumis de préférence à une incubation ou à plusieurs incubations pour multiplier le nombre de microorganismes dans ce résidu, et
d) le nombre et/ou la taille et/ou la nature des microorganismes est déterminé(e) dans le résidu.

2. Procédé selon la revendication 1, **caractérisé en ce que** la contamination microbienne est constituée de bactéries et/ou de champignons et/ou de levures.

3. Procédé selon l'une quelconque des revendications 1 ou 2, **caractérisé en ce que** la substance organique dégradable par des microorganismes est ajoutée sous la forme d'un milieu nutritif pour les microorganismes.

4. Procédé selon la revendication 3, **caractérisé en ce que** le milieu nutritif contient une source de carbone, d'azote, de phosphate et/ou de soufre et éventuellement, de minéraux, de substances de croissance et/ou de vitamines.

5. Procédé selon l'une ou plusieurs des revendications précédentes, **caractérisé en ce que** la centrifugation s'effectue à 100 - 1500 g, de préférence 200 - 1200 g, de manière très préférentielle à 600 - 1000 g.

6. Procédé selon l'une ou plusieurs des revendications précédentes, **caractérisé en ce que** la température d'incubation est de 20 à 37° C, de préférence de 28 à 34° C et de manière très préférentielle de 31,5 à 32,5° C.

7. Procédé selon l'une ou plusieurs des revendications précédentes, **caractérisé en ce que** l'on utilise les milieux nutritifs qui permettent sélectivement la croissance des germes à déterminer.

8. Procédé selon l'une ou plusieurs des revendications précédentes, **caractérisé en ce que** l'on utilise comme milieu, un bouillon de gélose tryptone azolectine/tween 20 (TAT), et/ou une solution de glucose et/ou de peptone/caséine et/ou un bouillon nutritif, de préférence de la gélose au soja (TSA).

9. Procédé selon l'une ou plusieurs des revendications précédentes, **caractérisé en ce que** la concentration du milieu nutritif pour les microorganismes est de préférence de 0,1 à 10 % en poids, de préférence de 2 à 5 % en poids, de manière très préférentielle de 3 % en poids.

10. Procédé selon l'une ou plusieurs des revendications précédentes, **caractérisé en ce que** la suspension, l'émulsion ou la dispersion contient en outre des polymères sous forme colloïdale.

11. Procédé selon l'une ou plusieurs des revendications précédentes, **caractérisé en ce que** la détermination qualitative et/ou quantitative des bactéries et/ou des champignons et/ou des levures s'effectue parallèlement dans une analyse.

12. Procédé selon l'une ou plusieurs des revendications précédentes, **caractérisé en ce que** l'étape de centrifugation s'effectue sur une durée de 1 à 30 min, de préférence de 2 à 15 minutes, de manière très préférentielle de 10 min.

13. Procédé selon l'une ou plusieurs des revendications précédentes, **caractérisé en ce que** la phase présentant les microorganismes, obtenue par centrifugation, est analysée par un procédé d'analyse de la taille des particules sur la quantité des microorganismes.

14. Procédé selon la revendication 13, **caractérisé en ce que** la taille et le nombre des microorganismes sont analysés, l'échantillon de microorganismes étant aspiré à l'aide d'un vide pratiqué au travers de pores de longueur et de diamètre définis, une tension existant entre l'entrée et la sortie des pores, et lors du passage des microorganismes dans les pores, une impulsion de courant qui est directement proportionnelle au volume du microorganisme étant mesurée et le nombre d'impulsion de courant étant proportionnel à la quantité de microorganismes.

15. Procédé selon l'une ou plusieurs des revendications précédentes, **caractérisé en ce que** la phase présentant des microorganismes, obtenue par centrifugation, est analysée par détermination de l'ATP produite par les microorganismes sur la quantité des microorganismes.

16. Procédé selon l'une ou plusieurs des revendications précédentes, **caractérisé en ce que** la contamination microbienne des dispersions, émulsions et suspensions est analysée dans l'industrie de transformation des métaux, dans l'industrie des pigments et du papier ainsi que dans les eaux de circulation de l'industrie du papier.
